# EUROPEAN PATENT APPLICATION

(11) **EP 1 574 825 A1**
(43) Date of publication of application: **14.09.2005**
(21) Application number: 04405147.2
(22) Date of filing: 12.03.2004
(51) Int. Cl.: G01D 5/30

(54) **Device for determining the longitudinal and angular position of a rotationally symmetrical apparatus**

(71) Applicant: Xitact S.A., 1110 Morges (CH)
(72) Inventor: Vecerina, Ivan, 1010 Lausanne (CH); Zoethout, Jurjen, 1530 Payerne (CH); Betrisey, Stéphane, 1110 Morges (CH)
(74) Representative: Richard, François-Régis

(57) **Abstract**

A device for determining the longitudinal and angular position of a rotationally symmetrical apparatus (3) when guided inside a longitudinal element surrounding the same where a spherical object is held in motion-transmitting contact with said apparatus, and including an imaging optical navigation sensor that measures the motion of the underlying surface of said spherical object by comparing successive images. Additional features of the captured images are used to identify the insertion or withdrawal of the instrument, as they cause a displacement of the bead, therefore allowing the determination of the absolute position of the instrument. The device comprises a light source (31) and a light detector (32). Light emitted by said light source (31) is directed onto a surface (101) of a spherical object (100) being in motion-transmitting contact with said rotationally symmetrical apparatus (3). Reflected light from said surface (101) is detected by said light detector (32) to produce a position signal showing a locally varying distribution in the longitudinal direction (110) and in the peripheral direction (112) to enable said precise position and angular measurement.

## Description

### Field of the invention

The present invention relates to a device for determining the longitudinal and angular position of a rotationally symmetrical apparatus used during simulated or real medical interventions, especially percutaneous interventions and especially a device for measuring the motion of a rotationally symmetric apparatus using an optical navigation sensor. It also relates to an optical navigation sensor for indirectly tracking the motion of the surface of an instrument as it passes through a static element that surrounds it, such as an insertion sheath. The present invention furthermore relates to techniques that allow the identification of an inserted instrument or the determination of its absolute position, and the tracking of particular instruments, such as wires of a small diameter.

### Background of the invention

Surgery simulators, as well computer assisted surgery systems and other applications, require or can benefit from a determination of the position of instruments manipulated by users. In minimally invasive or percutaneous procedures, where instruments usually pass through an insertion port such as a trocar or sheath, measuring the motion relative to such a surrounding structure can provide all or part of the information needed by a computer to either apply the effect of a user's gestures to a simulation model, or guide, assist or control the diagnostic or therapeutic procedure being performed. Other systems may rely on the same tracking device to otherwise process input from users who manipulate the tracked instruments, or to analyze the motion of the instruments.

Instrument tracking can either be based on absolute position measurements, or on continuous motion measurements starting from a known reference position. In the latter case, a means to establish the absolute position of the instrument is necessary initially, and eventually at regular intervals - to compensate the accumulation of measurement errors. An absolute position measurement can be established. Such an initial reference position can be established by requesting that the user places the system in a defined state, by detecting the insertion of the instrument, or by detecting absolute position markers on its surface (as disclosed by the inventors in WO 02/071369).

Prior art discloses devices for tracking the relative motion of instruments, catheters, or other elongated instruments using a mechanical system that is in contact with the moving instrument.

Some systems use tracking wheels or a similar mechanism directly driven by cables or gears attached to the moving instrument. This allows a reliable measurement of the instrument's motion, but typically demands that the moving instrument be attached to the tracking device, preventing users from easily and completely withdrawing the instrument from the device.

In US Patent 3,304,434 and US Patent 6,038,488 an arrangement is disclosed wherein a spherical object is in direct contact with the tracked surface; driven by friction, the sphere rolls in place to follow the motion of the underlying surface; the motion of the sphere itself is then measured by two linear motion encoders driven by a pair of shafts mounted in tangential engagement with the sphere, in a force-transmitting contact.

Each shaft then drives a coded wheel that reports the motion along an axis, providing tracking of each axis of motion.

Drawbacks of this approach include the friction imposed on the tracked instrument and the inertia of the mechanism, both of which can be felt by a user manipulating the tracked instrument. Also, slippage problems caused by an unreliable transmission of the instrument's motion to the spherical object can degrade the accuracy of the system. A spring-based mechanism is typically used to hold the bead in place, and further increases friction.

Other catheter-tracking devices, as described in EP 99108415.3 , rely on a carriage assembly for holding a catheter between a pair of opposed pinch wheels. The carriage assembly rotates to rotate the said catheter about its longitudinal axis, and the pinch wheels rotate to translate the object axially. The inertia of this mechanism, however, perceptibly interferes with the free motion of the catheter.

In another invention, disclosed in US 6.267,599, a framed assembly that includes rotation sensors is mounted on parallel guide rails. A motorized system ensures that this assembly follows the motion of the tip of an inserted instrument. Servo motors and force sensors are used to compensate for the friction and inertia of the system. Again, such systems fail to eliminate perceptible and disturbing haptic artifacts.

US 6,323,837 discloses another measurement method for tracking the angular position of a rod or catheter used within the simulation of surgical operations. To measure the motion of the instrument, it uses two independent and orthogonal interfaces, composed of a driving wheel that drives the motion of a coded wheel - which is preferably a black coded transparent wheel with optical encoders as transducers to sense its rotation. A problem of this approach is the same friction which is used to drive each interface generates a resistance to the motion of the instrument in the other, orthogonal direction.

Another approach is to use a tight grid-like or striped marking of the instrument's surface, as described in WO 98/10387. The device disclosed therein allows a contact-free reading of the motion of the instrument, but can only track specially designed surfaces. The tracked surface must be covered with a tight striped or grid-like pattern to allow motion detection. This increases manufacturing costs, and limits the type of instruments and surfaces that can be tracked. The surface coating is also often fragile: stains or scratches are likely to interfere with the tracking. Furthermore, the resolution that can be obtained with such a system is also limited.

The need remains, therefore, for a compact device that is able to conduct a precise measurement of the longitudinal and rotational position of an instrument without interfering with the manipulation of the instrument through excessive friction or inertia.

Optical tracking devices based on image capture and analysis, known as optical navigation sensors, have been introduced more recently. They have primarily been developed to improve the reliability and performance of computer mice (US 5,578,813, US 5,644,139, US 6,256,016, US 6,281,882). Unlike previous technologies which required a specific treatment or fabrication of the underlying surface (US 4,409,479), these optical navigation sensors capture consecutive images of a moving surface, and matching each newly acquired image with translated copies of previous images. This allows the sensors to analyze and precisely measure the motion of a nearby surface without requiring any physical contact, and allowing almost any type of surface to be tracked.

These sensors are used in particular to measure the displacement of devices along two orthogonal linear axes in a flat plane, as occurs within computer mice. The use of these sensors in different configurations have also been disclosed, for example to track the motion of a user's finger along 2 orthogonal axes (US 6,057,540), as part of surface image scanning devices (US 5,994,710), or within bar-code reading instruments (US 6,585,158).

### Summary of the invention

The present invention relates on the insight that optical navigation sensors, which are commonly found in so-called optical computer mice, can be used to indirectly track the motion of a rotationally symmetrical apparatus by tracking the surface of a small and lightweight spherical object held in motion-transmitting contact with the apparatus.

The set object is met in accordance with the invention by means of a device in accordance with the wording of claim 1 using an optical navigation sensor to indirectly track the longitudinal motion and the rotation around a longitudinal axis of a rotationally symmetrical instrument.

The features according to claim 1 allow a low-friction and low-inertia position determination using an indirect measurement. In comparison to a device that directly tracks the surface of the instrument, this device can effectively track a greater variety of surface materials, including dark, shiny, or transparent surfaces, as well as instruments of a very small diameter.

Compared to other devices that measure the rotation of a spherical object held in motion-transmitting contact with the instrument, this invention relies on an approach that minimizes friction and inertia, by using a small spherical object that is tracked and pressed against the instrument using contact-free approaches

Further preferred embodiments of the apparatus according to the invention are characterized in the dependent claims.

An apparatus for interfacing the movement of a rotationally symmetrical apparatus with a computer includes a support that allows two degrees-of-freedom, and includes and an optical navigation sensor. When a shaft is engaged with the support, it can move with two degrees of freedom while being held in contact with a motion-transmitting bead, where the optical navigation sensor senses each degree of freedom through the bead. The optical navigation sensor provides a simultaneous tracking of the combined translational and rotational displacement of the apparatus.

An intermediate sphere that is motion-transmitting contact with the instrument is inserted between the tracked object and the optical navigation sensor to alleviate limitations of the said sensor: when the instrument is excessively small relative to the size of the image captured by the sensor; or when the surface properties of the instrument (such as excessive shininess or high light absorption) are incompatible with an image capture of sufficient quality.

The invention enhances the tracking of rotationally symmetrical instruments that move through a surrounding structure - which may be a dedicated insertion port such as a trocar or sheath, or another surrounding piece that is used as a reference for position measurements.

While not totally contact-free, such a device generates a very low friction and inertia. Furthermore, the presence or absence of an inserted instrument can be detected through a displacement of the spherical object in or out of the image focus area.

Another object is to describe how information that is obtained from the processing of images captured by the optical navigation sensor can be used as a means to establish the absolute longitudinal and rotational position of the instrument: by detecting the presence or absence of an instrument within the tracking device, or by detecting optical markings on the surface of the instrument. Additionally, the detection of surface markings can be used to identify the instrument. A further object of the invention is therefore to provide the possibility to readily recognize the presence of a simulated instrument and/or to determine the kind of the present simulated instrument and/or its absolute/reference position within the tracking device.

Another object of the invention is to provide a device able to deliver information about the motion of an inner catheter being located inside an outer catheter or flexible element or instrument.

### Brief description of the drawings

- Fig. 1: shows the physical arrangement of an optical sensor and a spherical object ("the bead") above the surface to be tracked according to an embodiment of the invention,
- Fig. 2: shows the placement of a magnet used to improve the transmission of motion from the tracked surface to the intermediate bead,
- Fig. 3: shows how the displacement of the bead can be used to detect the presence or absence of an inserted apparatus, whose surface is to be tracked,
- Fig. 4: shows a mechanism that allows instruments of varying diameters to be tracked by the device according to the embodiment of Fig. 1,
- Fig. 5: shows examples of images captured by an optical navigation sensor used for motion-tracking, and
- Fig. 6: shows a schematic view of an instrument presenting a succession of colored segments, visible along the surface of an instrument.

### Detailed description of the invention

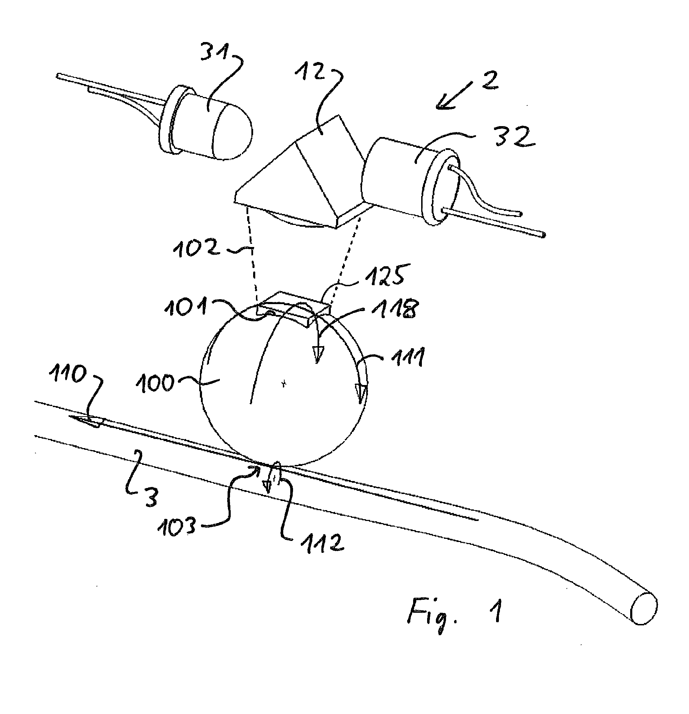

Fig. 1 shows a schematic view of a device using an optical navigation sensor 2 and a spherical object 100 (hereafter the "bead") used to transmit information about the motion of an underlying surface point 103.

An instrument-holding device (not shown) comprises a bearing (not shown) for a bead 100 and an opening or transparent area through which an optical navigation sensor 2 can track the intermediate underlying surface 101 of the bead 100, seen within the image capturing volume 125. The opening or transparent area within the instrument-holding device is at least as large as the maximum field of vision of the imaging optical navigation sensor 2, delimited by dotted lines 102. The bead 100 is mounted for free rotation about all axes: it is held in place with minimal friction, so that it cannot displace itself -- or so that only a motion away from the optical sensor 2 may be allowed, allowing the bead 100 to leave the intermediate image-capture zone 125.

The image capture volume 125 is delimited longitudinally by the depth of view defined by the optical element 12 and the relative position of the transducer 32. Surfaces that are closer to or further from the sensor 2 that the image capture volume will not be illuminated and imaged properly. Transversally, the image capture volume 125 is delimited by the area, within the depth of view, that is projected by optical element 12 onto the photosensitive area of transducer 32.

The instrument-holding device is connected with a refractive optical element 12 of the optical navigation sensor 2 in such a way that the bead 100, positioned underneath the sensor and in motion-transmitting contact with the instrument 3, is held at an adequate distance of the optical navigation sensor 2, so the surface 101 of the bead 100 remains in focus for the image capture used for instrument tracking.

The point 103 of motion-transmitting contact between the bead 100 and the surface to be tracked is diametrically opposed to the bead area tracked by the optical sensor 2. Therefore, the motion of the tracked surface 103 along the axes 110 and 112 is transmitted to the bead 100 by direct contact, and measured by the optical navigation sensor 2 as a displacement of the same magnitude along axes 111 and 118, respectively.

By using the bead 100 to capture and transmit the motion of the underlying object 3 to the optical sensor 2, this measuring unit can alleviate limitations of the optical navigation sensor and optical element 12. In particular:
- The navigation sensor may fail to capture usable images of some excessively smooth, excessively shiny, or excessively dark (light-absorbing) surfaces. The bead's surface and texture can be chosen to ensure adequate tracking, and proper motion transmission from the instrument's surface regardless of its optical properties.
- A tracked instrument may have an excessively small diameter, which is not large enough to be adequately imaged by the navigation sensor, or which is too small to cover its image-capture area 125. It is possible to correct this problem by developing a specially adapted optical element 12, or by inserting one or more lenses between the optical element 12 and the underlying surface. However, it can be preferable to use an intermediate bead of adequate diameter to capture and transmit the motion of the instrument to the optical navigation sensor.
   Specific implementations and features of this embodiment of the invention will be disclosed hereafter.

The correspondence of the surface motion recorded by the sensor and the instrument's motion is shown with arrows 110, 112, 111, 118. The surface motion axes reported by the optical navigation sensor 2, labeled with arrows 111 and 118, respectively measure the longitudinal translation and axial rotation of the bead.

The optical navigation sensor 2 comprises a light source 31 and a light-detecting and image-capturing transducer 32. The light source 31 can be a LED or another appropriate light emitting element. The transducer 32 can be a suitable array of photo detectors or e.g. a CCD device. The optical navigation sensor 2 optically detects motion by directly imaging as an array of pixels the various particular optical features visible in the image capture volume 125. The light from the light source 31 reflected from the surface 101 is focused onto the transducer 32 inside the optical navigation sensor 2. The responses of the individual photo detectors or CCD device are digitized to a suitable resolution and stored as a frame into corresponding locations within an array of memory. The image-capturing transducer 32 can be embedded within a chip that also includes a processor that processes the successive frames and continuously measures their relative displacement.

The 2-D optical navigation sensor 2 with integrated image processing can be composed of one of several existing devices, such as the ADNS-2001 or ADNS-2030 or ADNS-2051 manufactured by Agilent Technologies. This sensor based on the processing of a sequence of captured images is mounted behind refractive and lens system 12, which focuses an image-capture grid to a certain distance from the sensor itself. A light emitting diode 31 is located nearby to ensure adequate illumination of the surface underneath the sensor.

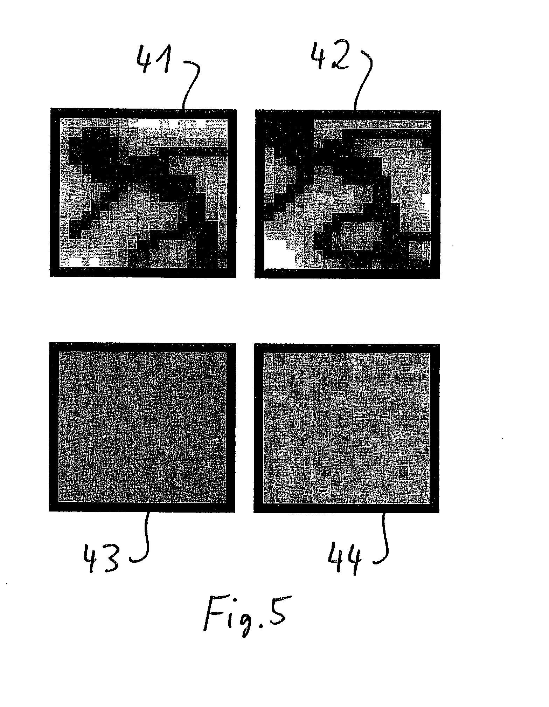

Fig. 5 shows examples of images captured by an optical navigation sensor for surface motion tracking, to illustrate how information is obtained from the images captured by the optical navigation sensor 2:
Images 41 and 42 show two consecutive images captured by the optical navigation sensor 2 used for tracking the surface of the bead. A comparison and matching of the initial image 41 (left) and the subsequent one 42 (right) allows to determine the magnitude and the direction of image displacement, which is proportional to the displacement of the tracked surface.

When the sensor is integrated in a device such as those described in this invention, this motion information can be translated into the longitudinal and rotational motion of the instrument 3: The image displacement is separated into a longitudinal component 111, parallel to the axis of the instrument 3, and a transverse component 118 orthogonal to the previous axis - and are scaled according to the resolution of the image and it scaling. The longitudinal component directly measures the longitudinal motion of the instrument through the tracking device; the transverse displacement of the instrument's surface is converted into a measure of the instrument's rotation relative to the tracking device, by dividing it by the radius of the instrument.

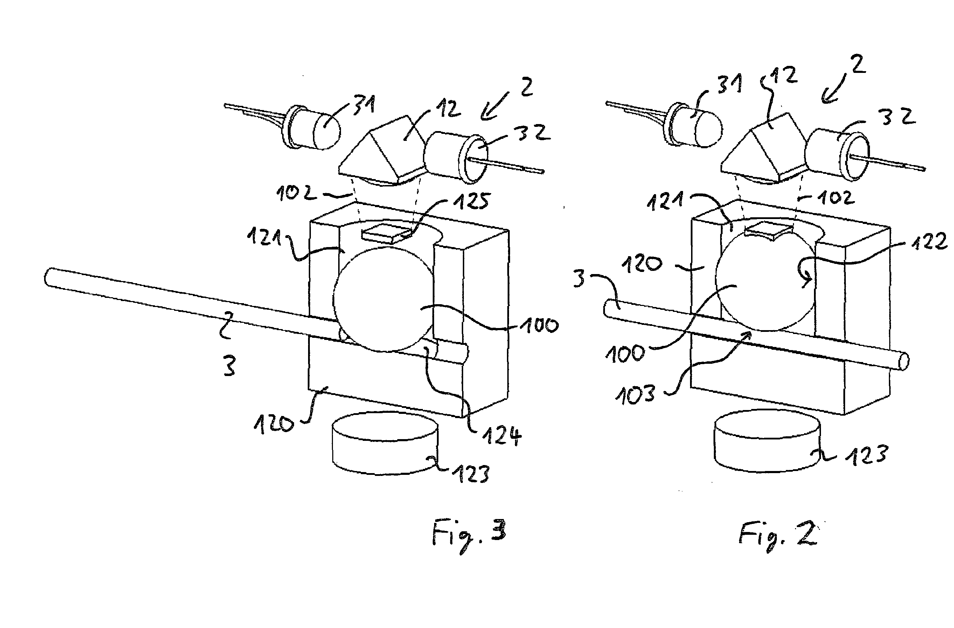

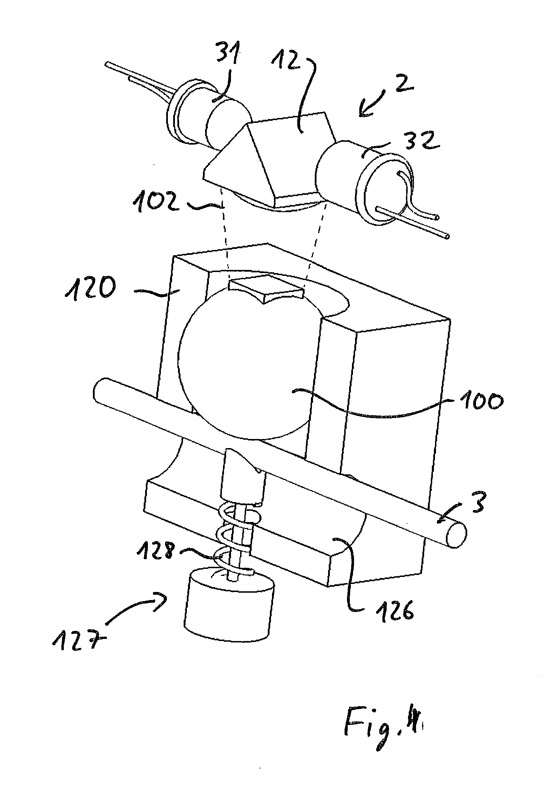

Images 43 and 44 show the difference that may typically be found between an image 43 (left) taken when no surface is visible in the image capture volume 125, and an image 44 (right) taken while the bead's surface has been brought in view within the image capture volume 125. The contrast between areas of the image increases in a way that can be detected. Under controlled and constant illumination, the luminosity of the image also typically increases. Fig. 2 and Fig 3 illustrate how the insertion of an instrument can displace the bead in and out of the image capture volume 125. This displacement of the bead can be detected when the sensor successively reports images similar to image 43 (=Fig 3: bead out, no instrument inserted) and to image 44 (=Fig 4: bead in, instrument is present).

In an embodiment of this invention that uses the ADNS-2051 optical navigation sensor, the following properties of the image captured by the optical navigation sensor are used: 1. A measure of the intensity of the light diffusely reflected by the surface, a.k.a. the brightness of the image (B). 2. The variability of the luminosity on different areas of the captured image, which is somewhat related to the contrast of the image (C). On the ADNS-2051, a measure of the contrast (C) is available as a surface quality measurement (SQUAL register), and the overall image brightness (B) can be estimated by dividing the average pixel value by the shutter time (Average_Pixel /Shutter_Lower or Shutter_Upper). An increase of these two values indicates the situation depicted in image 44 and Fig 4 (instrument inserted), while a decrease reflect the situation depicted in image 43 and Fig 3 (no instrument inserted).

The transition between these two states therefore allows the detection of the passage of the instrument's tip in the device, which lifts the bead upon entry, and lets it move away upon exit. When this transition occurs, the absolute position of the instrument's tip is known - and can serve as a reference to subsequently determine the absolute position of the instrument from displacement measurements that are reported by the ADNS-2051 processor.

Optionally, a small-area optical reflective sensor, such as the HEDS-1100 manufactured by Agilent Technologies, may be added to the device to measure the luminosity of a single point on the surface of the instrument itself with a high accuracy. Such an optical reflective sensor is a fully integrated module containing a LED emitter and a matched IC photo detector in one housing. A bifurcated aspheric lens is used to image the active areas of the emitter and the detector to one single spot. This device can be used to detect optical markings on the surface of the instrument, such as colored segments of distinct colors.

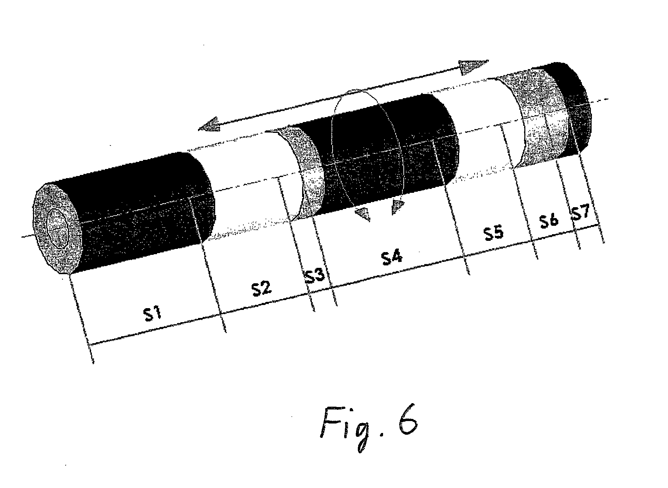

Fig. 6 shows a schematic view of an instrument 3 made of a succession of colored, shaded or differently textured segments, that may also be painted or engraved. While measuring the motion of the instrument, the separate small-area optical reflective sensor can be queried about the luminosity of the surface it is sensing. The distinctly shaded areas can therefore be detected as they pass underneath the imaging optical navigation sensor 2, and the length of each segment can be measured during the longitudinal motion of the instrument (in the direction 21 labeled with an X axis). The resulting pattern of segment colors and lengths (e.g. black-S1, white-S2, grey-S3, black-S4, white-S5, grey-S6 and black-S7) can be used to encode information. This information can be used as a unique signature for each instrument, thus providing for automated instrument identification as it is inserted, or to identify a specific area along the length of the instrument.

In particular, this information can be used to establish the absolute position of the tracked instrument within the device, as a transition between two colored segments uniquely identified by their shade or length is detected. This absolute position can serve as a reference to subsequently determine the absolute position of the instrument from displacement measurements that are reported by the ADNS-2051 processor.

In Fig. 1, the bead 100 can have a diameter below 1 cm, while the section of the inserted instrument 3 may have any diameter, going down to a fraction of a millimeter. For example, some guidewires used in medical procedures have a diameter of 0.3 mm, which is smaller than the area imaged by the optical navigation sensor and optical elements commonly found on the market.

Because the bead 100 has a small size and mass, its inertia remains very low, and the friction forces that are transmitted to the moving apparatus can remain imperceptible.

One or more optical navigation sensors 2 could be used to track the motion of the bead 100, and other sensor locations could be chosen, but tracking the bead motion in a location that is diametrically opposed to where the bead 100 is in contact with the tracked surface is optimal for an optical navigation sensor, and simplifies calculations. The longitudinal movement of the underlying object 3 is shown with arrow 110 being translated into the rotary movement 111 of the bead 100. The rotary movement of the underlying object 3 is shown with arrow 112 being translated into the rotary movement 118 of the bead 100.

Fig. 2 shows details of an embodiment according to Fig. 1 of the invention applied to the tracking of a rotationally symmetrical, small-diameter apparatus 3, such as a catheter or guidewire used in a real or simulated medical intervention.

The body 120 of the device (the front half of which has been removed in this view) is traversed by a tubular cavity, through which an instrument 3 can be inserted and moved freely. The bead 100 is located inside a cylindrical well 121 drilled within the body, perpendicular to said cavity guiding the apparatus 3.

An optical navigation sensor 2, attached to the top of the well 121, measures the rotation of the bead 100. As the motion of the instrument 3 drives the rotation of the bead 100, the surface displacement measured by the optical sensor 2 directly corresponds (same magnitude, but opposite direction) to the displacement of the instrument's surface at the point of contact 103 with the bead 100 - as described in Fig. 1.

For the bead 100 to reliably follow the instrument's motion, the friction force between the bead 100 and instrument at contact point 103 needs to be more important than the friction at the contact zone 122 between the bead 100 and the walls of the well 121. If the device is oriented so that the bead 100 is vertically above the catheter, gravity alone can ensure that the bead 100 moves with the instrument.

Alternatively, a force can be applied on the bead 100 to generate adequate contact pressure between the bead 100 and the moving instrument 3, because of the small mass and size of the bead 100. For example, a permanent magnet or electromagnet 123 can be used to passively or actively pull the bead 100 towards the instrument 3, provided that the bead 100 is of a paramagnetic material. This ensures that the bead 100 reliably follows the motion of the inserted instrument 3, and that the device can function in any orientation, independently of any gravity.

This magnetic system has a negligible impact on the friction and inertia of the tracking device.

Fig. 3, to be compared to Fig. 2, demonstrates how the device can detect the insertion of a catheter. When no instrument is inserted, as shown in Fig. 3, the bead 100 sits further from the optical sensor, pulled away by gravity or a mechanism such as the magnet 123 used to attract the bead 100 towards the instrument insertion cavity.

The bead 100 overlaps the instrument-insertion cavity 124, and is beyond the image capturing volume and focus depth 125 of the image capturing system embedded in the optical navigation sensor 2.

Fig. 2 shows the bead's position after the insertion of an instrument 3: the inserted instrument 3 has lifted the bead 100, which is now in-focus for adequate imaging and tracking by the optical navigation sensor 2.

Referring back to Fig. 5, image 43 and 44 reflect the picture that would be captured by the imaging sensor in the situation illustrated in Fig. 3 and Fig. 2, respectively.

As explained for the embodiment of Fig 1 and 5, the optical navigation sensor provides output signals that reflect the sharpness and quality of the image captured by the optical navigation sensor 2. The displacement of the bead 100, triggered by the insertion (Fig. 2) or withdrawal (Fig. 3) of an instrument 3, can therefore be detected reported by the tracking device.

Fig. 4 shows an embodiment of the invention that allows instruments 3 of various diameters to be inserted within the tracking device.

The tracking system presents the same components described in Fig. 2 and 3, but the instrument-insertion cavity 126 now includes a mechanism 127 that allows the tracking of a range of instrument diameters. The bottom of the cavity 126 now includes a mobile centering device 127 that pushes the inserted instrument 3 towards the optical sensor 2, with a force slightly higher than the pressure that the bead 100 exercises on an inserted instrument 3. The concave shape of the cavity bottom 126, on a section transversal to the axis of the instrument 3, ensures that an inserted catheter is properly centered, in addition to being lifted up against the tracking bead 100.

To allow the insertion of the instrument 3, the centering device 127 may be lifted by an active mechanism only when the system detects, by a separate means, that an instrument 3 has been inserted. Alternatively, the centering device 127 may be passively lifted by a spring-based 128 mechanism.

By measuring the position of the centering device 127, the diameter of an inserted instrument 3 can be determined. This diameter value is used in the computation of the instrument's rotation angle from the transversal displacement measured by the optical device -- as described above.

The device according to the Fig. 1 to 4 shows the application of the optical navigation sensor 2, combined with the use of a motion-transmitting bead 100, to the analysis of an instrument's motion through a surrounding static element 120. This device can track various rigid or flexible instruments 3, including catheters and guidewires, such as those used for medical interventions, as well as during procedures simulated for training.

The key benefits of the device according to Fig. 1 to 4 is its ability to track the longitudinal and rotational motion of rotationally symmetrical instruments 3 that have an arbitrary diameter, and an arbitrary surface quality, including very thin metallic guidewires that could directly be tracked by an optical navigation sensor 2. This is achieved while keeping the friction and inertia of the system very low, making them nearly imperceptible to a user manipulating an inserted instrument. It has also been demonstrated that the presence or absence of an inserted instrument can also be established through the displacement of the bead.

Another embodiment not shown in the Fig. and providing information about the longitudinal and rotational motion of an e.g. outer catheter and an inner catheter provided in the outer catheter is using the teaching of this application. Said rotationally symmetrical apparatus 3 is a transparent first rotationally symmetrical apparatus 3. The transparency is to be provided in all parts which may be subject (following a longitudinal and rotational motion of the catheter 3) to pass in vicinity of the optical navigation sensor. Inside said first apparatus 3 is a second inner rotationally symmetrical apparatus, which can be seen from outside the first apparatus 3. Therefore a second light source and a second image-capturing and processing device is provided, wherein light emitted by said second light source is directed directly on the outer surface of the second rotationally symmetrical apparatus. The surface equivalent to surface 101 is in this case on the inner catheter. Reflected light from said surface is detected by said second light detector to produce a position signal showing a locally varying distribution in the longitudinal direction and in the peripheral direction to enable a relative position and angular measurement for the inner catheter. This embodiment necessitates a greater diameter of the inner catheter so said the image surface of the second light source has sufficient dimensions to allow the measurement, even through the transparent outer device. In this embodiment the bead 100 is not only used to allow the magnification of the image but to provide a reflective surface, the surface of the outer catheter itself being transparent.

It is also possible to provide two or more devices according to Fig. 1 at different longitudinal places of the apparatus 3. It is then preferred to use a control unit connected to the output of both sensors. In this way possible slippage occurring at one navigation sensor can be detected and corrected.

## Claims

1. A device for determining the longitudinal (110) and angular (112) position of a rotationally symmetrical apparatus (3) when moving inside a surrounding element (120), **characterized in that** it comprises at least one light source and at least one light detector, wherein light emitted by said light source is directed on the outer surface (101) of a spherical object (100), said spherical object (100) being in motion-transmitting contact with said rotationally symmetrical apparatus (3), wherein reflected light from said surface (101) is detected by said light detector to produce a position signal showing a locally varying distribution in the longitudinal direction and in the peripheral direction to enable a relative position (110) and angular (112) measurement.

2. A device for determining the longitudinal (110) and angular (112) position of a rotationally symmetrical apparatus (3) when guided inside a surrounding element (120), **characterized in that** it comprises at least one light source and an image-capturing and processing device using reflected light (101) from a spherical object (100) being in motion-transmitting contact with the apparatus (3), wherein the image displacement information provided by the optical navigation sensor (2) is used to compute the longitudinal (110) and rotational (112) motion of the rotationally symmetrical apparatus (3).

3. The device according to claim 1 or claim 2, **characterized in that** the rotationally symmetrical apparatus (3) is an instrument (3) used in a real or simulated medical intervention.

4. The device according to one of claims 1 to 3, **characterized in that** it further comprises an optical element (12) to focus the emitted light (102) onto said surface (101).

5. The device according to one of claims 1 to 4, **characterized in that** it comprises at least one light source and an image-capturing and processing device, combined with at least one single-point optical sensor to measure the luminosity of a single point on the surface of the instrument with an increased accuracy.

6. The device according to one of claims 1 to 5, **characterized in that** a means to produce a magnetic or electrostatic field (123) is provided to attract or repel the spherical object (100) onto the rotationally symmetrical apparatus (3) or that the bead (100) is mounted above the rotationally symmetrical apparatus (3) to use gravity to attract the spherical object (100) onto the rotationally symmetrical apparatus (3).

7. The device according to one of claims 1 to 6, **characterized in that** the bearing (120, 124) of the spherical object (100) enables a displacement of the spherical object (100) upon insertion of the rotationally symmetrical apparatus (3) and wherein a light detector is positioned to detect said displacement.

8. The device according to one of claims 1 to 7, **characterized in that** the device further comprises means to accommodate rotationally symmetrical apparatus (3) of different diameters (126, 128), means (127, 128) to detect the diameter of the rotationally symmetrical apparatus (3) and calculating means to compute this diameter information into the angular rotation (112) of the rotationally symmetrical apparatus (3) based on its transversal surface motion.

9. The device according to one of claims 1 to 8, **characterized in that** said rotationally symmetrical apparatus (3) is a transparent first rotationally symmetrical apparatus (3) and **in that** said first apparatus (3) comprises a second inner rotationally symmetrical apparatus and **in that** at least a second light source and a second image-capturing and processing device is provided, wherein the image displacement information provided by the second optical navigation sensor is used to compute the longitudinal and rotational motion of said second apparatus.
